# EUROPÄISCHE PATENTSCHRIFT

(11) **EP 2 294 206 B1**
(45) Veröffentlichungstag und Bekanntmachung des Hinweises auf die Patenterteilung: **03.06.2015**
(21) Anmeldenummer: 09772206.0
(22) Anmeldetag: 29.04.2009
(51) Int. Cl.: C12P 7/40, C12P 7/42, C07C 51/02, B01D 11/04

(54) **Verfahren zur Herstellung freier alpha- oder beta-Hydroxycarbonsäuren**
Method for the production of free alpha- or beta-hydroxycarboxylic acids
Procédé de production d'acides alpha- ou beta-hydroxycarboxyliques libres

(30) Priorität: 04.07.2008 DE 102008040193
(43) Veröffentlichungstag der Anmeldung: 16.03.2011
(73) Patentinhaber: Evonik Röhm GmbH, 64293 Darmstadt (DE)
(72) Erfinder: HAAS, Thomas, 48161 Münster (DE); TACKE, Thomas, 63755 Alzenau (DE); MARX, Achim, 63571 Gelnhausen (DE); SCHRAVEN, Alexander, 47661 Issum (DE); ZEHNACKER, Olivier, 45657 Recklinghausen (DE); WITTMANN, Eva-Maria, Traunreut 83301 (DE)
(86) Internationale Anmeldenummer: PCT/EP2009/055163
(87) Internationale Veröffentlichungsnummer: WO 2010/000506

(56) Entgegenhaltungen:
- EP-A- 0 359 043
- WO-A-00/14052
- US-A- 4 405 717
- US-A- 4 444 881
- WASEWAR K L ET AL: "Fermentation of glucose to lactic acid coupled with reactive extraction: a review" INDUSTRIAL & ENGINEERING CHEMISTRY RESEARCH, Bd. 43, Nr. 19, 15. September 2004 (2004-09-15), Seiten 5969-5982, XP002559880
- CHEN RONGFU ET AL: "Membrane-mediated extractive fermentation for lactic acid production from cellulosic biomass" APPLIED BIOCHEMISTRY AND BIOTECHNOLOGY, HUMANA PRESS, INC, US, Bd. 63-65, 1. Januar 1997 (1997-01-01), Seiten 435-448, XP008087560 ISSN: 0273-2289
- LEWIS VIVIAN P ET AL: "Novel extractive fermentation process for propionic acid production from whey lactose" BIOTECHNOLOGY PROGRESS 1992 MAR-APR, Bd. 8, Nr. 2, März 1992 (1992-03), Seiten 104-110, XP002559991
- DATABASE WPI Week 200744 Thomson Scientific, London, GB; AN 2007-451202 XP002559992 & JP 2007 082490 A (TOYOTA CHUO KENKYUSHO KK) 5. April 2007 (2007-04-05)
- JAN MARTAK ET AL: "Toxicity of organic solvents used in situ in fermentation of lactic acid by Rhizopus arrhizus" BIOTECHNOLOGY TECHNIQUES, KLUWER ACADEMIC PUBLISHERS, DO, Bd. 11, Nr. 2, 1. Februar 1997 (1997-02-01), Seiten 71-75, XP019232506 ISSN: 1573-6784
- KESHAV A ET AL: "Extraction of propionic acid with tri-n-octyl amine in different diluents" SEPARATION AND PURIFICATION TECHNOLOGY, ELSEVIER SCIENCE, AMSTERDAM, NL, Bd. 63, Nr. 1, 20. April 2008 (2008-04-20), Seiten 179-183, XP024528103 ISSN: 1383-5866 [gefunden am 2008-04-20]
- ALI DEMIRCI ET AL: "Rapid screening of solvents and carrier compounds for lactic acid recovery by emulsion liquid extraction and toxicity on Lactobacillus casei (ATCC 11443)" BIOSEPARATION, KLUWER ACADEMIC PUBLISHERS, DO, Bd. 7, Nr. 6, 1. November 1998 (1998-11-01), Seiten 297-308, XP019232354 ISSN: 1573-8272

### SEQUENCE LISTING

<110> Evonik Röhm GmbH
<120> Verfahren zur Herstellung freier Carbonsäuren
<130> 2008P00197
<160> 1
<170> PatentIn version 3.4
<210> 1
   <211> 7296
   <212> DNA
   <213> Artificial
<220>
   <223> Plasmid
<400> 1
   accttcggga gcgcctgaag cccgttctgg acgccctggg gccgttgaat cgggatatgc     60
   aggccaaggc cgccgcgatc atcaaggccg tgggcgaaaa gctgctgacg gaacagcggg    120
   aagtccagcg ccagaaacag gcccagcgcc agcaggaacg cgggcgcgca catttccccg    180
   aaaagtgcca cctgggatga atgtcagcta ctgggctatc tggacaaggg aaaacgcaag    240
   cgcaaagaga aagcaggtag cttgcagtgg gcttacatgg cgatagctag actgggcggt    300
   tttatggaca gcaagcgaac cggaattgcc agctggggcg ccctctggta aggttgggaa    360
   gccctgcaaa gtaaactgga tggctttctt gccgccaagg atctgatggc gcaggggatc    420
   aagatctgat caagagacag gatgaggatc gtttcgcatg attgaacaag atggattgca    480
   cgcaggttct ccggccgctt gggtggagag gctattcggc tatgactggg cacaacagac    540
   aatcggctgc tctgatgccg ccgtgttccg gctgtcagcg caggggcgcc cggttctttt    600
   tgtcaagacc gacctgtccg gtgccctgaa tgaactgcag gacgaggcag cgcggctatc    660
   gtggctggcc acgacgggcg ttccttgcgc agctgtgctc gacgttgtca ctgaagcggg    720
   aagggactgg ctgctattgg gcgaagtgcc ggggcaggat ctcctgtcat ctcaccttgc    780
   tcctgccgag aaagtatcca tcatggctga tgcaatgcgg cggctgcata cgcttgatcc    840
   ggctacctgc ccattcgacc accaagcgaa acatcgcatc gagcgagcac gtactcggat    900
   ggaagccggt cttgtcgatc aggatgatct ggacgaagag catcaggggc tcgcgccagc    960
   cgaactgttc gccaggctca aggcgcgcat gcccgacggc gaggatctcg tcgtgaccca   1020
   tggcgatgcc tgcttgccga atatcatggt ggaaaatggc cgcttttctg gattcatcga   1080
   ctgtggccgg ctgggtgtgg cggaccgcta tcaggacata gcgttggcta cccgtgatat   1140
   tgctgaagag cttggcggcg aatgggctga ccgcttcctc gtgctttacg gtatcgccgc   1200
   tcccgattcg cagcgcatcg ccttctatcg ccttcttgac gagttcttct gagcgggact   1260
   ctggggttcg aaatgaccga ccaagcgacg cccaacctgc catcacgaga tttcgattcc   1320
   accgccgcct tctatgaaag gttgggcttc ggaatcgttt tccgggacgc cggctggatg   1380
   atcctccagc gcggggatct catgctggag ttcttcgccc acccccatgg gcaaatatta   1440
   tacgcaaggc gacaaggtgc tgatgccgct ggcgattcag gttcatcatg ccgtttgtga   1500
   tggcttccat gtcggcagaa tgcttaatga attacaacag tttttatgca tgcgcccaat   1560
   acgcaaaccg cctctccccg cgcgttggcc gattcattaa tgcagctggc acgacaggtt   1620
   tcccgactgg aaagcgggca gtgagcgcaa cgcaattaat gtgagttagc tcactcatta   1680
   ggcaccccag gctttacact ttatgcttcc ggctcgtatg ttgtgtggaa ttgtgagcgg   1740
   ataacaattt cacacaggaa acagctatga ccatgattac gccaagcgcg caattaaccc   1800
   tcactaaagg gaacaaaagc tgggtaccgg gccccccctc gaggtcgacg gtatcgataa   1860
   gcttgatatc gaattcctgc agcccggggg atccactagt tctagaaata attttgttta   1920
   actttaagaa ggaattcagg agcccttcac catgacctgg cttgagccgc agataaagtc   1980
   ccaactccaa tcggagcgca aggactggga agcgaacgaa gtcggcgcct tcttgaagaa   2040
   ggcccccgag cgcaaggagc agttccacac gatcggggac ttcccggtcc agcgcaccta   2100
   caccgctgcc gacatcgccg acacgccgct ggaggacatc ggtcttccgg ggcgctaccc   2160
   gttcacgcgc gggccctacc cgacgatgta ccgcagccgc acctggacga tgcgccagat   2220
   cgccggcttc ggcaccggcg aggacaccaa caagcgcttc aagtatctga tcgcgcaggg   2280
   ccagaccggc atctccaccg acttcgacat gcccacgctg atgggctacg actccgacca   2340
   cccgatgagc gacggcgagg tcggccgcga gggcgtggcg atcgacacgc tggccgacat   2400
   ggaggcgctg ctggccgaca tcgacctcga gaagatctcg gtctcgttca cgatcaaccc   2460
   gagcgcctgg atcctgctcg cgatgtacgt ggcgctcggc gagaagcgcg gctacgacct   2520
   gaacaagctg tcgggcacgg tgcaggccga catcctgaag gagtacatgg cgcagaagga   2580
   gtacatctac ccgatcgcgc cgtcggtgcg catcgtgcgc gacatcatca cctacagcgc   2640
   gaagaacctg aagcgctaca acccgatcaa catctcgggc taccacatca gcgaggccgg   2700
   ctcctcgccg ctccaggagg cggccttcac gctggccaac ctgatcacct acgtgaacga   2760
   ggtgacgaag accggtatgc acgtcgacga attcgcgccg cggttggcct tcttcttcgt   2820
   gtcgcaaggt gacttcttcg aggaggtcgc gaagttccgc gccctgcgcc gctgctacgc   2880
   gaagatcatg aaggagcgct tcggtgcaag aaatccggaa tcgatgcggt tgcgcttcca   2940
   ctgtcagacc gcggcggcga cgctgaccaa gccgcagtac atggtcaacg tcgtgcgtac   3000
   gtcgctgcag gcgctgtcgg ccgtgctcgg cggcgcgcag tcgctgcaca ccaacggcta   3060
   cgacgaagcc ttcgcgatcc cgaccgagga tgcgatgaag atggcgctgc gcacgcagca 3120
   gatcattgcc gaggagagtg gtgtcgccga cgtgatcgac ccgctgggtg gcagctacta 3180
   cgtcgaggcg ctgaccaccg agtacgagaa gaagatcttc gagatcctcg aggaagtcga 3240
   gaagcgcggt ggcaccatca agctgatcga gcagggctgg ttccagaagc agattgcgga 3300
   cttcgcttac gagaccgcgc tgcgcaagca gtccggccag aagccggtga tcggggtgaa 3360
   ccgcttcgtc gagaacgaag aggacgtcaa gatcgagatc cacccgtacg acaacacgac 3420
   ggccgaacgc cagatttccc gcacgcgccg cgttcgcgcc gagcgcgacg aggccaaggt 3480
   gcaagcgatg ctcgaccaac tggtggctgt cgccaaggac gagtcccaga acctgatgcc 3540
   gctgaccatc gaactggtga aggccggcgc aacgatgggg gacatcgtcg agaagctgaa 3600
   ggggatctgg ggtacctacc gcgagacgcc ggtcttctga gcaggaagct tcccaccatg 3660
   gaccaaatcc cgatccgcgt tcttctcgcc aaagtcggcc tcgacggcca tgaccgaggc 3720
   gtcaaggtcg tcgctcgcgc gctgcgcgac gccggcatgg acgtcatcta ctccggcctt 3780
   catcgcacgc ccgaagaggt ggtcaacacc gccatccagg aagacgtgga cgtgctgggt 3840
   gtaagcctcc tgtccggcgt gcagctcacg gtcttcccca agatcttcaa gctcctggac 3900
   gagagaggcg ctggcgactt gatcgtgatc gccggtggcg tgatgccgga cgaggacgcc 3960
   gcggccatcc gcaagctcgg cgtgcgcgag gtgctactgc aggacacgcc cccgcaggcc 4020
   atcatcgact cgatccgctc cttggtcgcc gcgcgcggcg cccgctgaaa gggcgagctc 4080
   tccaattcgc cctatagtga gtcgtattac gcgcgctcac tggccgtcgt tttacaacgt 4140
   cgtgactggg aaaaccctgg cgttacccaa cttaatcgcc ttgcagcaca tccccctttc 4200
   gccagctggc gtaatagcga agaggcccgc accgatcgcc cttcccaaca gttgcgcagc 4260
   ctgaatggcg aatggaaatt gtaagcgtta atattttgtt aaaattcgcg ttaaattttt 4320
   gttaaatcag ctcatttttt aaccaatagg ccgactgcga tgagtggcag ggcggggcgt 4380
   aattttttta aggcagttat tggtgccctt aaacgcctgg tgctacgcct gaataagtga 4440
   taataagcgg atgaatggca gaaattcgaa agcaaattcg acccggtcgt cggttcaggg 4500
   cagggtcgtt aaatagccgc ttatgtctat tgctggttta ccggtttatt gactaccgga 4560
   agcagtgtga ccgtgtgctt ctcaaatgcc tgaggccagt ttgctcaggc tctccccgtg 4620
   gaggtaataa ttgacgatat gatcatttat tctgcctccc agagcctgat aaaaacggtg 4680
   aatccgttag cgaggtgccg ccggcttcca ttcaggtcga ggtggcccgg ctccatgcac 4740
   cgcgacgcaa cgcggggagg cagacaaggt atagggcggc gaggcggcta cagccgatag 4800
   tctggaacag cgcacttacg ggttgctgcg caacccaagt gctaccggcg cggcagcgtg 4860
   acccgtgtcg gcggctccaa cggctcgcca tcgtccagaa aacacggctc atcgggcatc 4920
   ggcaggcgct gctgcccgcg ccgttcccat tcctccgttt cggtcaaggc tggcaggtct 4980
   ggttccatgc ccggaatgcc gggctggctg ggcggctcct cgccggggcc ggtcggtagt 5040
   tgctgctcgc ccggatacag ggtcgggatg cggcgcaggt cgccatgccc caacagcgat 5100
   tcgtcctggt cgtcgtgatc aaccaccacg gcggcactga acaccgacag gcgcaactgg 5160
   tcgcggggct ggccccacgc cacgcggtca ttgaccacgt aggccgacac ggtgccgggg 5220
   ccgttgagct tcacgacgga gatccagcgc tcggccacca agtccttgac tgcgtattgg 5280
   accgtccgca aagaacgtcc gatgagcttg gaaagtgtct tctggctgac caccacggcg 5340
   ttctggtggc ccatctgcgc cacgaggtga tgcagcagca ttgccgccgt gggtttcctc 5400
   gcaataagcc cggcccacgc ctcatgcgct ttgcgttccg tttgcaccca gtgaccgggc 5460
   ttgttcttgg cttgaatgcc gatttctctg gactgcgtgg ccatgcttat ctccatgcgg 5520
   tagggtgccg cacggttgcg gcaccatgcg caatcagctg caacttttcg gcagcgcgac 5580
   aacaattatg cgttgcgtaa aagtggcagt caattacaga ttttctttaa cctacgcaat 5640
   gagctattgc ggggggtgcc gcaatgagct gttgcgtacc cccctttttt aagttgttga 5700
   tttttaagtc tttcgcattt cgccctatat ctagttcttt ggtgcccaaa gaagggcacc 5760
   cctgcggggt tcccccacgc cttcggcgcg gctccccctc cggcaaaaag tggcccctcc 5820
   ggggcttgtt gatcgactgc gcggccttcg gccttgccca aggtggcgct gcccccttgg 5880
   aacccccgca ctcgccgccg tgaggctcgg ggggcaggcg ggcgggcttc gccttcgact 5940
   gcccccactc gcataggctt gggtcgttcc aggcgcgtca aggccaagcc gctgcgcggt 6000
   cgctgcgcga gccttgaccc gccttccact tggtgtccaa ccggcaagcg aagcgcgcag 6060
   gccgcaggcc ggaggctttt ccccagagaa aattaaaaaa attgatgggg caaggccgca 6120
   ggccgcgcag ttggagccgg tgggtatgtg gtcgaaggct gggtagccgg tgggcaatcc 6180
   ctgtggtcaa gctcgtgggc aggcgcagcc tgtccatcag cttgtccagc agggttgtcc 6240
   acgggccgag cgaagcgagc cagccggtgg ccgctcgcgg ccatcgtcca catatccacg 6300
   ggctggcaag ggagcgcagc gaccgcgcag ggcgaagccc ggagagcaag cccgtagggc 6360
   gccgcagccg ccgtaggcgg tcacgacttt gcgaagcaaa gtctagtgag tatactcaag 6420
   cattgagtgg cccgccggag gcaccgcctt gcgctgcccc cgtcgagccg gttggacacc 6480
   aaaagggagg ggcaggcatg gcggcatacg cgatcatgcg atgcaagaag ctggcgaaaa 6540
   tgggcaacgt ggcggccagt ctcaagcacg cctaccgcga gcgcgagacg cccaacgctg 6600
   acgccagcag gacgccagag aacgagcact gggcggccag cagcaccgat gaagcgatgg 6660
   gccgactgcg cgagttgctg ccagagaagc ggcgcaagga cgctgtgttg gcggtcgagt 6720
   acgtcatgac ggccagcccg gaatggtgga agtcggccag ccaagaacag caggcggcgt   6780
   tcttcgagaa ggcgcacaag tggctggcgg acaagtacgg ggcggatcgc atcgtgacgg   6840
   ccagcatcca ccgtgacgaa accagcccgc acatgaccgc gttcgtggtg ccgctgacgc   6900
   aggacggcag gctgtcggcc aaggagttca tcggcaacaa agcgcagatg acccgcgacc   6960
   agaccacgtt tgcggccgct gtggccgatc tagggctgca acggggcatc gagggcagca   7020
   aggcacgtca cacgcgcatt caggcgttct acgaggccct ggagcggcca ccagtgggcc   7080
   acgtcaccat cagcccgcaa gcggtcgagc cacgcgccta tgcaccgcag ggattggccg   7140
   aaaagctggg aatctcaaag cgcgttgaga cgccggaagc cgtggccgac cggctgacaa   7200
   aagcggttcg gcaggggtat gagcctgccc tacaggccgc cgcaggagcg cgtgagatgc   7260
   gcaagaaggc cgatcaagcc caagagacgg cccgag                             7296

## Beschreibung

### Gebiet der Erfindung

Gegenstand der Erfindung ist ein Verfahren zur Herstellung von freien α- oder β-Hydroxycarbonsäuren umfassend die Verfahrensschritte
A) Herstellung von α- oder β-Hydroxycarbonsäure durch eine sich in einem wässrigen Medium befindende, biologische Zelle, welche ein Mikroorganismus ist, unter Zugabe eines Amins der allgemeinen Formel (I) 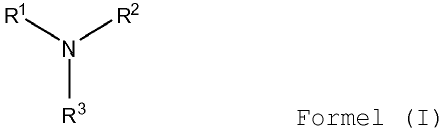 wobei R¹, R² und R³ unabhängig voneinander gleiche oder ungleiche, verzweigte oder unverzweigte, gegebenenfalls substituierte Kohlenwasserstoffreste oder H darstellt,
B) für den Fall, dass das zugegebene Amin in Verfahrensschritt A) wasserlöslich ist, Zugabe eines wasserunlöslichen Amins der allgemeinen Formel (I),
   wobei in dem Verfahrensschritt A) beziehungsweise B) ein Mehrphasensystem erhalten wird und sich aus dem wasserunlöslichen Amin und der α- oder β-Hydroxycarbonsäure das korrespondierende Ammoniumcarboxylat bildet, und
C) Abtrennen der wasserunlöslichen Phase und
D) Erhitzen der wasserunlöslichen Phase unter Freisetzung von freier α- oder β-Hydroxycarbonsäure, , dadurch gekennzeichnet, dass die α- oder β-Hydroxycarbonsäure ausgewählt ist aus der Gruppe bestehend aus Milchsäure, Zitronensäure, Weinsäure, Glykolsäure, 2-Hydroxyisobuttersäure, 3-Hydroxypropionsäure, 3-Hydroxybuttersäure, 3-Hydroxyvaleriansäure, 3- Hydroxyhexansäure, 3-Hydroxyheptansäure, 3-Hydroxyoctansäure und 3-Hydroxyisobuttersäure, wobei der Begriff "wasserunlöslich" als eine Löslichkeit von weniger als 100g/kg wässriger Lösung und der Begriff "wasserlöslich" als eine Löslichkeit von gleich oder mehr als 100g/kg wässriger Lösung definiert ist.

### Stand Technik

Die biochemische Herstellung von Carbonsäuren ist aufgrund von z.B. der Milch- oder Zitronensäureproduktion gut bekannt. Da die meisten Fermentationsprozesse bei einem pH-Wert des Mediums durchgeführt werden, der oberhalb des pK_{S}-Wertes der herzustellenden Carbonsäure liegt, fallen die Carbonsäuren zum Grossteil als Salz und nicht als freie Säure an. Diese Carbonate werden meist durch Zugabe von Säuren in ihre freien Säuren überführt.

WO9815517 beschreibt ein Verfahren zur Extraktion von Milchsäure mit einem basischen, organischem Lösungsmittel bzw. wasserunmischbaren Aminen.

DE 102006052311 beschreibt ein Verfahren zur Herstellung freier α-Hydroxycarbonsäuren durch Erhitzen der korrespondierenden Ammoniumcarboxylate in Gegenwart von tertiären Aminen unter destillativer Entfernung des entstehenden Ammoniaks gefolgt von weiterer Abdestillation und damit einhergehender Bildung des tertiären Amins und der freien α-Hydroxycarbonsäure.

US 4,275,234 beschreibt ein extraktives Verfahren von Carbonsäuren mit Aminen als Extraktionsmittel umfassend einen zusätzlichen, wässrigen Rückextraktionsschritt, der die Carbonsäure wieder in wässriger Lösung vorliegen lässt.

US 4,444,881 beschreibt ein Verfahren zur Isolierung von organischen Säuren aus Fermentationsbrühe durch Überführung der Säure in ihr Calciumsalz, Beimengung eines wasserlöslichen tertiären Amin-Carbonates unter Bildung des Trialkylammoniumsalzes und präzipitierendem Calciumcarbonat, Aufkonzentrierung der Trialkylammoniumsalzlösung und Spaltung des Trialkylammoniumsalzes durch Erhitzen.

EP 1385593 beschreibt ein Verfahren zur Aufarbeitung von kurzkettigen Carbonsäuren aus einer Lösung ihrer Alkylammoniumkomplexen durch Destillation unter Zugabe eines azeotrophierenden Kohlenwasserstoffes bei Bedingungen, unter denen der Alkylammoniumkomplex in die freie kurzkettige Carbonsäure und das Alkylamin zerfällt.

US 5,510,526 beschreibt ein Verfahren zur Aufarbeitung von freier Milchsäure aus einer Fermentationsbrühe durch Extraktion mit einem Extraktionsmittel beinhaltend ein nicht mit Wasser mischbares Trialkylamin mit einer Anzahl von mindestens 18 Kohlenstoffatomen in Gegenwart von CO₂, Abtrennung der organischen von der wässrigen Phase und abschließender Trennung der freien Milchsäure von der organischen Phase.

WO02090312 beschreibt einen Prozess zur Aufreinigung von freien Carbonsäuren aus wässrigen Lösungen, bei dem die wässrige Lösung als Mischung mit einem organischen Lösungsmittel erhitzt wird und so die freie Säure erhalten wird.

US 5,132,456 beschreibt ein mehrstufiges Verfahren zur Aufreinigung von freien Carbonsäuren aus einem wässrigen Medium, bei dem zunächst die Carbonsäure mit einem Säure absorbierendem Mittel extrahiert wird und nach Trennung dieses Mittels von dem wässrigen Medium aus/von diesem Säure absorbierendem Mittel mit wasserlöslichen Aminen die Carbonsäure erneut als Ammoniumcarboxylat rückextrahiert wird. Anschließend wird das Ammoniumcarboxylat gespalten.
Der Übersichtsartikel von Wasewar K L et al. (Ind. Eng. Chem. Res. 2004, vol. 43, p. 5969-5982) beschreibt die Fermentation und reaktive Extraktion von Milchsäure, mit Schwerpunkt auf die Extraktion mit Alkylaminen. Es werden verschiedene Methoden zur Isolierung der Milchsäure aus der organischen Phase beschrieben, vornehmlich mittels Rückextraktion in eine wässrige Phase.

Allen Verfahren ist zum Nachteil, dass große Mengen an wässrigen Stoffströmen anfallen oder Produkte entstehen, die dem Verfahren nicht wieder zugeführt werden können und somit als Abfall verbleiben.
Aufgabe der Erfindung war es, ein Verfahren bereitzustellen, welches die vorgenannten Nachteile des Standes der Technik überwindet.

### Beschreibung Erfindung

Überraschenderweise wurde gefunden, dass ein Verfahren gemäß Anspruch 1 zur Herstellung von freien α- oder β-Hydroxycarbonsäuren umfassend die Verfahrensschritte Herstellung von α- oder β-Hydroxycarbonsäure durch eine biologische Zelle unter Zugabe eines Amins und gegebenenfalls für den Fall, dass dieses zugegebene Amin wasserlöslich ist, Zugabe eines weiteren, wasserunlöslichen Amins, Trennen der wasserunlöslichen Phase und Erhitzen derselben unter Freisetzung von freier α- oder β-Hydroxycarbonsäure die Aufgabe der Erfindung löst.
Vorteil der Erfindung ist es, dass eine Ansäuerung der Reaktionslösungen mit beispielsweise Mineralsäuren oder Kohlendioxid entfällt. Hierdurch entfällt das Anfallen von großen Salzmengen wie z.B. Ammoniumsulfat oder Gips, wie es oft in anderen Prozessen auftritt.
Ein weiterer Vorteil ist es, dass Wasser früh im Verfahren energetisch günstig, durch beispielweise eine Phasentrennung, abgetrennt wird und somit weiter energieverbrauchende Stoffströme im Verfahren reduziert werden. Ebenso ist es nicht notwendig diese Stoffströme zu Verdampfen.
Noch ein Vorteil der vorliegenden Erfindung ist die Tatsache, dass die bei biochemischen Prozessen oft auftretende Produktinhibierung durch Verwenden eines nicht wasserlöslichen Amines vermieden werden kann.

Der Begriff "Carbonsäure" im Sinne der vorliegenden Erfindung beinhaltet sowohl die freie Carbonsäure (-COOH) als auch das korrespondierende Salz (-COO⁻).

Der Begriff "Hydroxycarbonsäure" im Sinne der vorliegenden Erfindung beschreibt Carbonsäuren mit mindestens einer Hydroxyl- und einer Carbonsäuregruppe und beinhaltet sowohl die freie Carbonsäure (-COOH) als auch das korrespondierende Salz (-COO⁻).

Der Begriff "Ammoniumcarboxylat" im Sinne der vorliegenden Erfindung beinhaltet sämtliche Carboxylate einer einwertig positiv geladenen Gruppe mit einem vierbindigen Stickstoff. Als Beispiel sei folgende allgemeine Formel genannt 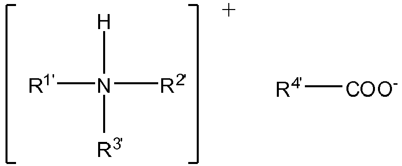 wobei R^{1'}, R^{2'}, R^{3'} und R^{4'} unabhängig voneinander gleiche oder ungleiche, verzweigte oder unverzweigte, gegebenenfalls substituierte Kohlenwasserstoffreste oder H darstellt.
Der Begriff "wasserunlöslich" im Sinne der vorliegenden Erfindung ist definiert als eine Löslichkeit von weniger als 100g/kg wässriger Lösung.
Der Begriff "wasserlöslich" im Sinne der vorliegenden Erfindung ist definiert als eine Löslichkeit von gleich oder mehr als 100g/kg wässriger Lösung.
Alle angegebenen Prozent (%) sind wenn nicht anders angegeben Massenprozent.
Gegenstand der Erfindung ist daher ein Verfahren zur Herstellung von freien α- oder β-Hydroxycarbonsäuren umfassend die Verfahrensschritte
A) Herstellung von α- oder β-Hydroxycarbonsäure durch eine sich in einem wässrigen Medium befindende, biologische Zelle, welche ein Mikroorganismus ist, unter Zugabe eines Amins der allgemeinen Formel (I) 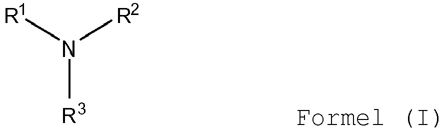 wobei R¹, R² und R³ unabhängig voneinander gleiche oder ungleiche, verzweigte oder unverzweigte, gegebenenfalls substituierte Kohlenwasserstoffreste oder H darstellt,
B) für den Fall, dass das zugegebene Amin in Verfahrensschritt A) wasserlöslich ist, Zugabe eines wasserunlöslichen Amins der allgemeinen Formel (I),
   wobei in dem Verfahrensschritt A) beziehungsweise B) ein Mehrphasensystem erhalten wird und sich aus dem wasserunlöslichen Amin und der α- oder β-Hydroxycarbonsäure das korrespondierende Ammoniumcarboxylat bildet, und
C) Abtrennen der wasserunlöslichen Phase und
D) Erhitzen der wasserunlöslichen Phase unter Freisetzung von freier α- oder β-Hydroxycarbonsäure, dadurch gekennzeichnet, dass die α- oder β-Hydroxycarbonsäure ausgewählt ist aus der Gruppe bestehend aus Milchsäure, Zitronensäure, Weinsäure, Glykolsäure, 2-Hydroxyisobuttersäure, 3-Hydroxypropionsäure, 3-Hydroxybuttersäure, 3-Hydroxyvaleriansäure, 3- Hydroxyhexansäure, 3-Hydroxyheptansäure, 3-Hydroxyoctansäure und 3-Hydroxyisobuttersäure, wobei der Begriff "wasserunlöslich" als eine Löslichkeit von weniger als 100g/kg wässriger Lösung und der Begriff "wasserlöslich" als eine Löslichkeit von gleich oder mehr als 100g/kg wässriger Lösung definiert ist.

In Verfahrensschritt A werden α- oder β-Hydroxycarbonsäuren durch eine sich in einem wässrigen Medium befindende, biologische Zelle, welche ein Mikroorganismus ist, nach dem Fachmann bekannten Verfahren hergestellt. Je nach eingesetzter Zelle und hergestellter Carbonsäure werden die Verfahrensparameter entsprechend angepasst. Bei diesen Verfahren kann es sich beispielsweise um fermentative Verfahren handeln.

Bevorzugt ist die eingesetzte Zelle ausgewählt aus der Gruppe der Gattungen umfassend:
*Aspergillus, Corynebacterium, Brevibacterium, Bacillus, Acinetobacter, Alcaligenes, Lactobacillus, Paracoccus, Lactococcus, Candida, Pichia, Hansenula, Kluyveromyces, Saccharomyces, Escherichia, Zymomonas, Yarrowia, Methylobacterium, Ralstonia, Pseudomonas, Rhodospirillum, Rhodobacter, Burkholderia, Clostridium und Cupriavidus.*

Besonders bevorzugt ist die eingesetzte Zelle ausgewählt aus der Gruppe umfassend *Aspergillus nidulans, Aspergillus niger, Alcaligenes latus, Bacillus megaterium, Bacillus subtilis, Brevibacterium flavum, Brevibacterium lactofermentum, Escherichia coli, Saccharomyces cerevisiae, Kluveromyces lactis, Candida blankii, Candida rugosa, Corynebacterium glutamicum, Corynebacterium efficiens, Zymomonas mobilis, Yarrowia lipolytica, Hansenula polymorpha, Methylobacterium extorquens, Ralstonia eutropha, insbesondere Ralstonia eutropha H16, Rhodospirillum rubrum, Rhodobacter sphaeroides, Paracoccus versutus, Pseudomonas aeruginosa, Pseudomonas putida, Acinetobacter calcoaceticus* und *Pichia pastoris,* wobei *Escherichia coli, Yarrowia lipolytica, Corynebacterium glutamicum und Ralstonia eutropha* ganz besonders bevorzugt sind.

Eine besondere Ausgestaltung erfindungsgemäßen Verfahrens zeichnet sich dadurch aus, dass in Verfahrensschritt A) eine Zelle eingesetzt wird, die aus mindestens einer Kohlenstoffquelle ausgewählt aus der Gruppe bestehend aus Kohlendioxid und Kohlenmonoxid α- oder β-Hydroxycarbonsäuren zu bilden vermag.

Somit handelt es sich in diesem Fall um den Einsatz acetogen bzw. autotroph wachsender. Zellen, bevorzugt um solche ausgewählt aus der Gruppe umfassend *Acetogenium kivui, Acetobacterium woodii, Acetoanaerobium noterae, Clostridium Aceticum, Butyribacterium methylotrophicum, Clostridium acetobutylicum, Clostridium thermoaceticum, Eubacterium limosum, Peptostreptococcus productus, Clostridium ljungdahlii, Ralstonia eutropha* und *Clostridium carboxydivorans.*

Als zugegebenes Amin in Verfahrensschritt A) können Ammoniak und Alkylamine eingesetzt werden; hierbei können primäre, sekundäre und tertiäre Alkylamine sowie quaternäre Aminsalze eingesetzt werden.

Es ist bevorzugt, dass das zugegegebene Amin in Verfahrensschritt A) eingesetzt wird, um den pH-Wert des wässrigen Mediums zu beeinflussen, bevorzugt ihn zu erhöhen. Bevorzugt wird der pH-Wert durch das zugegebene Amin in Verfahrensschritt A) in einem Bereich von bis 2 bis 9, bevorzugt von 4 bis 8, besonders bevorzugt von 5 bis 7 gehalten.

Bevorzugt werden als Amine in Verfahrensschritt A) wasserunlösliche Amine eingesetzt. Bevorzugt werden Amine in Verfahrensschritt A) eingesetzt bei denen R¹, R² und R³ unabhängig voneinander gleiche oder ungleiche, unverzweigte, unsubstituierte Alkylreste mit bevorzugt 2 bis 20, besonders bevorzugt 4 bis 16, ganz besonders bevorzugt 6 bis 12 C-Atomen, oder H darstellt.
Bevorzugt handelt es sich in Verfahrensschritt A) bei wasserunlöslichen Aminen um Alkylamine mit mindestens 16 C-Atomen, bevorzugt um Trialkylamine und besonders bevorzugt um Trialkylamine ausgewählt aus der Gruppe umfassend Trihexylamine, Trioctylamin, Tridecylamin, Tricaprylamine, Tridodecylamine.

In besonderen Ausführungsformen erfindungsgemäßen Verfahrens kann es vorteilhaft sein, in Verfahrensschritt A) Amine mit einer größeren Basenstärke einzusetzen; in diesem Fall ist es bevorzugt, dass als Amine Dialkylamine und bevorzugt Dialkylamine ausgewählt aus der Gruppe umfassend Di-Iso-Tridecylamin, Bis(2-ethylhexyl)Amin, Lauryl-Trialkyl-Methylamine, Diundecylamin, Didecylamin eingesetzt werden.

Das Amin wird in Verfahrensschritt A) mindesten im molaren Verhältnis von 1,1 zu 1, bevorzugt von 1,5 bis 5 zu 1 bezogen auf die Carbonsäure zugegeben.

Nach oder auch ständig während Verfahrensschritt A) können die flüssigen Anteilen oder ein Teil der flüssigen Anteile nach dem Fachmann bekannten Verfahren wie beispielsweise Zentrifugation, Tangentialfiltration etc. und die biologischen Zellen voneinander getrennt werden. Gegebenenfalls können abgetrennte Komponenten wie wässriges Medium oder Zellen dem Verfahren neu zugeführt werden.
Gegebenenfalls können die in Verfahrensschritt A) erhaltenen flüssigen Komponenten vor den weiteren Verfahrensschritten aufkonzentriert werden

Als zugegebenes Amin in Verfahrensschritt B) können sämtliche dem Fachmann bekannten, wasserunlöslichen Amine, bevorzugt Alkylamine eingesetzt werden. Bevorzugt werden wasserunlösliche Amine in Verfahrensschritt B) eingesetzt bei denen R¹, R² und R³ unabhängig voneinander gleiche oder ungleiche, unverzweigte, unsubstituierte Alkylreste mit bevorzugt 2 bis 20, besonders bevorzugt 4 bis 16, ganz besonders bevorzugt 6 bis 12 C-Atomen, oder H darstellt.

Weiter bevorzugt eingesetzte Amine entsprechen den oben genannten wasserunlöslichen Aminen in Verfahrensschritt A)

Das Amin wird in Verfahrensschritt B) mindesten im molaren Verhältnis von 1,1 zu 1, bevorzugt von 1,5 bis 5 zu 1 bezogen auf die α- oder β-Hydroxycarbonsäure zugegeben.

In Verfahrensschritt B) kann das wasserlösliche Amin des Verfahrensschrittes A) mindestens teilweise entfernt werden. Besonders, wenn in Verfahrensschritt B) ein wasserunlösliches Amin eingesetzt wird, welches eine schwächere Base darstellt (einen kleineren pK_{B} aufweist) als das wasserlösliche Amin, ist es bevorzugt, dass mindestens ein Teil des wasserlöslichen Amins entfernt wird.

Dies kann beispielsweise durch eine Extraktion, einen Ionentauscher, durch Erhitzen und somit thermisches Austreiben des wasserlöslichen Amins erreicht werden oder aber durch Einleitung von CO₂, wobei das wasserlösliche Amin als Carbonat ausfällt. Ein beispielhaftes Verfahren zur Abtrennung des wasserlöslichen Amins ist in der DE 102006052311 beschrieben. Das entfernte wasserlösliche Amin kann dem Verfahrensschritt A) erneut zugeführt werden.

Nach Verfahrensschritt A) beziehungsweise B) liegt ein Mehrphasensystem vor, und aus dem wasserunlöslichen Amin und der α- oder β-Hydroxycarbonsäure hat sich das korrespondierende Ammoniumcarboxylat gebildet.

Das Ammoniumcarboxylat liegt bevorzugt - bezogen auf gleiche Volumina von wässrigen zu organischen Phasen - zum Grossteil, bevorzugt zu mehr als 60%, besonders bevorzugt zu mehr als 80% und ganz besonders bevorzugt zu mehr als 90% in einer wasserunlöslichen Phase vor.

Ein zusätzliches, wasserunlösliches Lösungsmittel kann dem wasserunlöslichen Amin gegebenenfalls zugesetzt werden, um vorteilhafte Eigenschaften wie beispielsweise bessere Phasentrennungen oder stabilere Phasen zu gewährleisten, die Viskosität des Amins zu verringern oder die Löslichkeit der Carboxylsäure zu erhöhen.
Als zusätzliches, wasserunlösliches Lösungsmittel können beispielsweise Alkohole mit mindestens acht Kohlenstoffatomen, Ketone wie beispielsweise Methylisobutylketon, aromatische Lösungsmittel wie beispielsweise Toluol und Xylol, aliphatische unpolare Lösungsmittel wie beispielsweise Kerosin und Hexan eingesetzt werden. Bevorzugt werden Oleylalkohol und Dodecanol eingesetzt.
Bevorzugt werden solche zusätzlichen Lösungsmittel eingesetzt, dessen Siedepunkt oberhalb des Siedepunktes des wasserunlöslichen Amins liegt.

In Verfahrensschritt C) werden wasserunlösliche Phasen enthaltend das Ammoniumcarboxylat von der wässrigen Phase abgetrennt. Dies kann durch sämtliche dem Fachmann bekannte Verfahren erfolgen, mit denen man organische von wässrigen Phasen trennen kann, wie Beispielsweise Dekantation, Zentrifugation oder auch Destillation. Beispiele hierfür finden sich unter anderem in Perry's Chemical Engineers' Handbook (Section 15); By Robert H Perry, Don W Green, James O Malone; Published 1999; McGraw-Hill.

Im Rahmen des erfindungsgemäßen Verfahrens kann es vorteilhaft sein, dass die abgetrennte wässrige Phase dem Verfahren wieder zugeführt wird.
Ebenso kann es vorteilhaft sein, die abgetrennte wasserunlösliche Phase weiter zu reinigen, wie beispielsweise durch Extraktion, Filtration, Zentrifugation Ionentauschern oder Aufzukonzentrieren wie beispielsweise durch Destillation, Extraktion
Die Freisetzung von freier α- oder β-Hydroxycarbonsäure in Verfahrensschritt D) erfolgt durch Erhitzen der wasserunlöslichen Phase, bevorzugt unter vermindertem Druck, durch die Spaltung des Ammoniumcarboxylates.
Ein verminderter Druck bedeutet im Sinne der Erfindung einen Druck von weniger als 1*10⁵ Pa, bevorzugt weniger als 0.9*10⁵ Pa. und besonders bevorzugt weniger als 0,8*10⁵ Pa.
Die Art des Erhitzens ist abhängig von der verwendeten Apparatur/Anlage und kann beispielsweise über ein Heizbad, einen temperierbaren Reaktormantel oder durch Kontaktieren der wasserunlöslichen Phase mit einem erhitzten Gasstrom erfolgen. Die Temperatur wird in Abhängigkeit von dem verwandten Druck so gewählt, dass die thermische Salzspaltung stattfindet und die Bildung von Nebenprodukten minimiert wird. Bevorzugt wird gleichzeitig mindestens ein Teil der während der Reaktion gebildeten α- oder β-Hydroxycarbonsäure destillativ entfernt. Geeignete Temperatur- und Druckbereiche können von einem Fachmann bestimmt werden ebenso wie die notwendige Dauer der thermischen Behandlung beispielsweise durch ein Überwachen der gebildeten Amin- bzw. Carbonsäuremenge oder des Temperaturverlaufs der Reaktionslösung.
In einer bevorzugten Ausführungsform liegt die Temperatur in Verfahrensschritt D) in einem Temperaturbereich von 80°C bis 300°C, bevorzugt von 120°C bis 250°C, besonders bevorzugt von 150°C bis 220°C.
Das in Verfahrensschritt D) erhaltene wasserunlösliche Amin kann wieder dem Verfahren zugeführt werden.
Die in Verfahrensschritt D) erhaltene Produktfraktion enthaltend α- oder β-Hydroxycarbonsäuren kann ohne weitere Reinigung zu Folgeprodukten umgesetzt werden. Bevorzugt ist beispielsweise die Dehydratisierung von Hydroxycarbonsäuren zu ungesättigten Carbonsäuren.
Eine Reihe von Verfahren zur Dehydratisierung von Hydroxycarbonsäuren sind dem Fachmann bekannt, solche werden beispielsweise in der PCT/EP2007/055394, (veröffentlicht als WO2007/141208), US 3,666,805 und US 5,225,594 beschrieben
Das erfindungsgemäße Verfahren kann weiterhin eine oder mehrere anschließende Schritte zur Reinigung und Isolierung der Carbonsäuren aus der Produktfraktion enthalten. Geeignete Verfahrensschritte sind unter anderen Konzentrierung, Kristallisation, Ionenaustauschchromatographie, Elektrodialyse, Extraktion mit reaktiven Lösungsmitteln und die Reinigung durch Veresterung der Carbonsäure mit geeigneten Alkoholen, nachfolgender Destillation des erhaltenen Esters und anschließender Hydrolyse des Esters zur freien Säure sowie Kombinationen dieser Schritte. In der Produktfraktion enthaltene Nebenprodukte können vor oder nach der Isolierung der bei der thermischen Salzspaltung gebildeten freien Carbonsäure entfernt oder zur Carbonsäure umgesetzt werden.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Folgende Figuren sind Bestandteil der Beispiele:
Figur 1: Wachstum von Zellen in Anwesenheit von verschiedenen TOA-Konzentrationen
Figur 2: Herstellung von 2-Hydroxyisobuttersäure in Anwesenheit von verschiedenen TOA-Konzentrationen
Figur 3: Prozessskizze mit Einsatz eines wasserunlöslichen Amins in Verfahrensschritt A)
Figur 4: Prozessskizze mit Einsatz eines wasserlöslichen Amins in Verfahrensschritt A)

### Beispiele:

*Biokompatibilität von TOA (Trioctylamin) mit Ralstonia eutropha* Kulturen von Ralstonia *eutropha* PHB-4 (reklassifiziert als *Cupriavidus necator,* DSMZ 541) transformiert mit dem Plasmid: pBBR1MCS-2::*icmA*-*icmB* mit SEQ ID No 1 wurden im 400 ml Maßstab in einem Standardmedium 46 Stunden bis zu einer OD (optische Dichte bei 600 nm) von ca. 30, das entspricht einer CFU (colony formed units) von 1x10¹¹ pro ml, unter Standardbedingungen (30°C; pH 6,8; rpm 250-750; pO₂ 20%) fermentiert.
Nach 48 Stunden Fermentationszeit wurden 0,5%, 1%, 5% und 10% (w/v) Trioctylamin (TOA) steril zugegeben. Nach weiteren 6 und 20 Stunden wurden die CFU bestimmt.
Für die Ermittlung der CFU wurden Verdünnungsreihen der Brühen hergestellt. Die Verdünnungen wurden auf Agarplatten ausplattiert und 24 Stunden bei 30°C inkubiert. Es erfolgte eine vierfach Bestimmung der Verdünnungen 10⁷ und 10⁸.
Ergebnis: Die CFU nahm über einen Zeitraum von 20 Stunden (Fermentationszeit Stunde 46-65) in Anwesenheit von TOA (0,5%, 1%, 5% und 10% (w/v)) weiter zu.
Die Werte sind in der folgenden Tabelle zusammengefasst, die CFU Werte sind auf 1 ml Fermentationsbrühe normiert.

| | 0h | 6h | 24h |
|---|---|---|---|
| 0,5% TOA | 3,75E+10 | 5,31E+10 | 6,00E+10 |
| 1% TOA | 3,60E+10 | 3,73E+10 | 1,44E+11 |
| 5% TOA | 1,44E+11 | 1,25E+11 | 8,08E+11 |
| 10% TOA | 1,34E+11 | 1,61E+11 | 1,82E+11 |

### Herstellung von Carbonsäure durch eine sich in einem wässrigen Medium befindende, biologische Zelle in Anwesenheit eines Trialkylamins

2-Hydroxyisobuttersäure produzierende *Ralstonia eutropha* PHB-4 (reklassifiziert als *Cupriavidus necator,* DSMZ 541), welche mit dem Plasmid pBBRIMCS-2::*icmA*-*icmB*, Seq ID No 1, transformiert wurden, wurden als zwei 50 ml Kulturen in einem Schüttelinkubator unter Standardbedingungen (30°C, 140rpm , 20h) in LB Medium angezogen.
Davon wurden zur Biomasseproduktion 5 x 10 ml in 40 ml modifiziertes Mineralsalzmedium (nach Schlegel et al., 1961) überimpft und für 10h unter Standardbedingungen inkubiert.

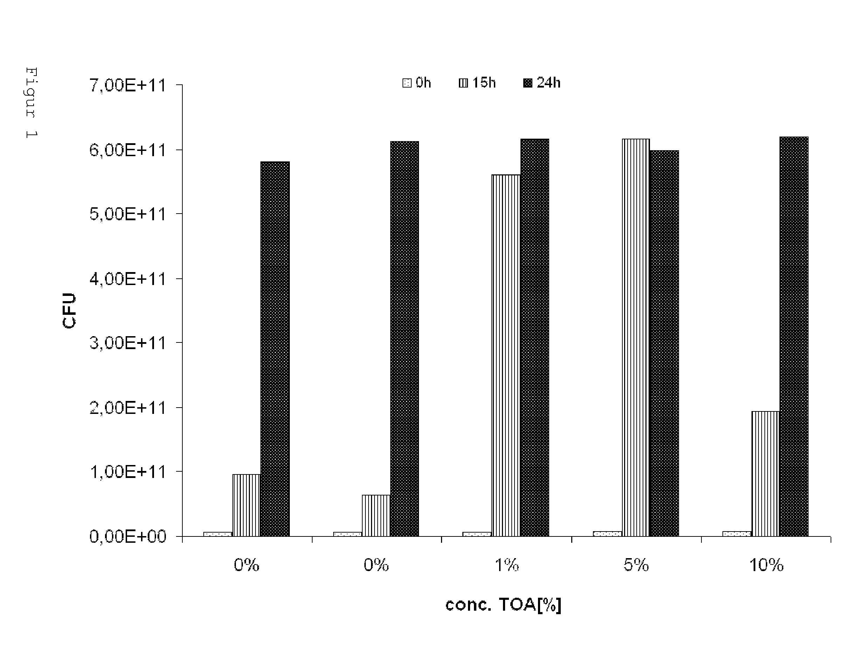

Die Biomassekulturen wurden abzentrifugiert und in 10 ml modifiziertem Mineralsalzmedium resuspendiert.
Mit den Resuspensionen wurden 5 Kolben eines Ramos Schüttelinkubator (Respiration Activity Monitoring System) angeimpft. Die Kolben waren mit modifizierten Mineralsalzmedium (Zusatz von 1g/L Hefeextrakt und 15g/L Fruktose) mit Vitamin B12 (60mg/l) befüllt. 3 Kolben enthielten zusätzlich 1, 5 und 10% (w/v) Trioctylamin (TOA).
Die Kolben wurden 24h unter Standardbedingungen (30°C, 140rpm) inkubiert.
Nach 0, 15 und 24 Stunden wurden Proben genommen und CFU (OD), pH, und 2HIB-Konzentration in der Brühe bestimmt.
Für die CFU Bestimmung wurden Verdünnungsreihen der Brühen hergestellt. Die Verdünnungen wurden auf LB- Agarplatten mit 300mg/l Kanamycin ausplattiert und 24h bei 30°C inkubiert. Es erfolgte eine 4fach Bestimmung der Verdünnungen 10⁻⁸ und 10⁻⁹. Die Figur 1 zeigt die Ergebnisse der Kultivierungen, CFU-Werte sind normiert auf 1 ml Fermentationsbrühe. Das Zellwachstum wird offensichtlich nicht durch die Anwesenheit des Alkylamins beeinflusst.
Die Bestimmung der Konzentration der hergestellten Carbonsäure 2-Hydroxyisobuttersäure erfolgte mittels Ionenchromatographie (IC(Methrom 761 Compact mit Autosampler, Methode Dionex AS154 x 250mm + Vorsäule AG 154 x 350ml) und HPLC (Agilent Technologies HPLC series 1200, Methode Aminex).

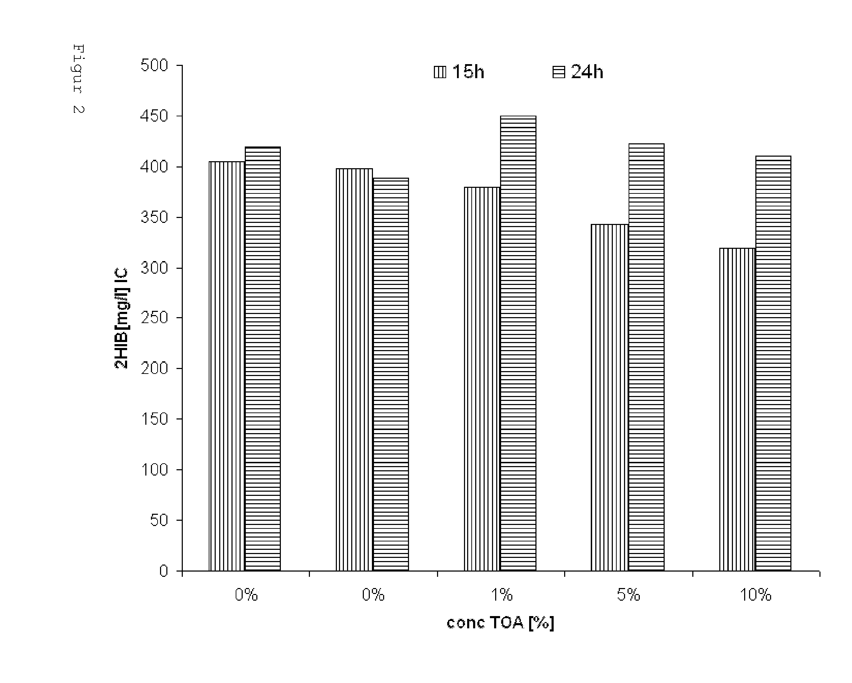

Es zeigt die Figur 2 die IC Messwert oben genannter Kultivierungen der 2-HIB-Konzentrationen.
In allen Ansätzen wird über den gesamten Versuchszeitaum hinweg 2-Hydroxyisobuttersäure gebildet.

### Thermisches Spalten eines Alkylammonium-Salzes

Eine Trioctylammoniumsalzlösung wurde durch Mischen von 2-Hydroxyisobuttersäure (2-HIB) mit Trioctylamin (TOA) hergestellt. Hierzu wurden 10 g 2-Hydroxyisobuttersäure eingewogen und in 90 g TOA gelöst. Aus dieser Einwaage ergibt sich ein stöchiometrisches Verhältnis TOA/2-Hydroxyisobuttersäure von 2,64.
In einem Rotationsverdampfer wurden 50,6 g dieser Trioctylammoniumsalzlösung vorgelegt. Mittels einer Vakuumpumpe wurde für die thermische Salzspaltung ein Absolutdruck von 27 mbar eingestellt. Das verwendete Ölbad für die Temperierung der Vorlage wurde auf 180 °C eingestellt und konstant gehalten. Nach etwa 20 min kristallisierten an den kühlen Glasteilen weiße Kristalle aus. Nach ca. 4 h wurde der Versuch abgebrochen. Das Auswiegen der Vorlage nach der thermischen Spaltung ergab eine Massenreduzierung von 3,5 g. Die am Glas befindlichen, weißen Kristalle wurden mit Wasser ausgewaschen und mittels HPLC analysiert. Diese Analyse zeigte, dass es sich bei diesen Kristallen um 2-Hydroxyisobuttersäure handelt. Mittels einer Elementaranalyse (C,H,N,O) wurden die Proben sowohl vor Versuchsbeginn, als auch nach Beendigung des Experimentes analysiert. Auf Basis dieser Analysen konnten die Konzentrationen und die Stoffmengen der jeweiligen Substanzen berechnet werden. 98,4 % der aufgegebene TOA Menge befanden sich nach dem Versuch noch in der Vorlage; 34,4 % der anfänglichen Stoffmasse der 2-Hydroxyisobuttersäure befanden sich zum Versuchsende noch als Trialkylammoniumsalz in der Vorlage. 65,6 % der eingewogenen 2-Hydroxyisobuttersäure Masse konnte thermisch vom Salz gespalten werden und teilweise als Kristalle an den kühlen Glasteilen zurückgewonnen werden.

### Beispielhafte Prozessskizze mit Einsatz eines wasserunlöslichen Amins in Verfahrensschritt A)

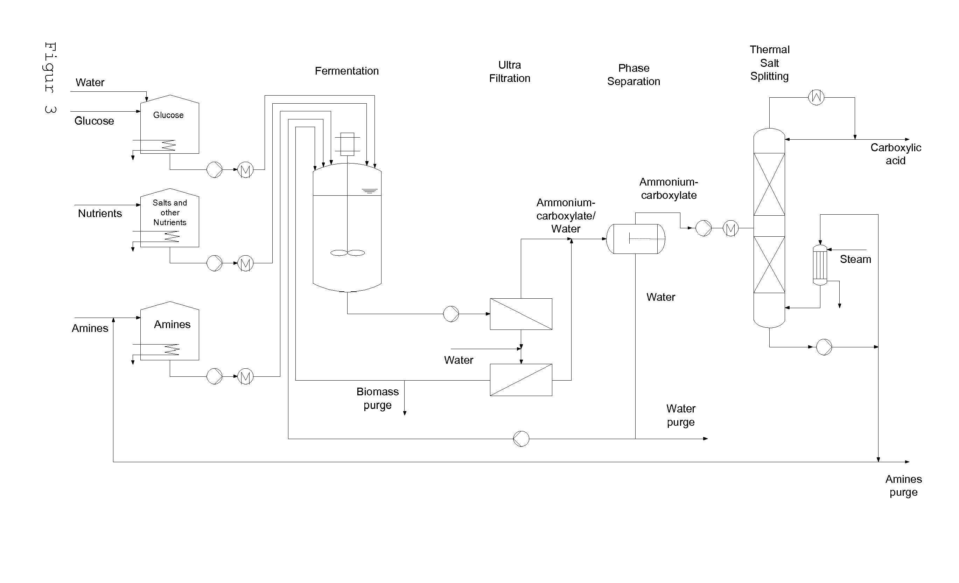

Figur 3 beschreibt eine Ausgestaltung des erfindungsgemäßen Verfahrens, bei welcher in Verfahrensschritt A) ein wasserunlösliches Amin eingesetzt wird. Diese werden in den Fermenter zur pH-Wert Regulierung eingesetzt. Hierdurch werden in dem Fermenter Ammoniumcarboxylate gebildet. Nach der Biomasseabtrennung können die wasserunlösliche Phase und die wässrige Phase separiert werden. Die wässrige Phase kann nach einer eventuellen Aufreinigung in den Fermenter zurückgeführt werden. In der wasserunlöslichen Phase befinden sich die Ammoniumcarboxylate. Diese können in einer nachgeschalteten thermischen Salzspaltung in die freie Säure und das korrespondierende Amin gespalten werden. Nach einer eventuellen Aufreinigung kann das Amin wieder in den Fermenter zur pH-Wert Regulierung zurück und somit im Kreis geführt werden.

### Beispielhafte Prozessskizze mit Einsatz eines wasserlöslichen Amins in Verfahrensschritt A)

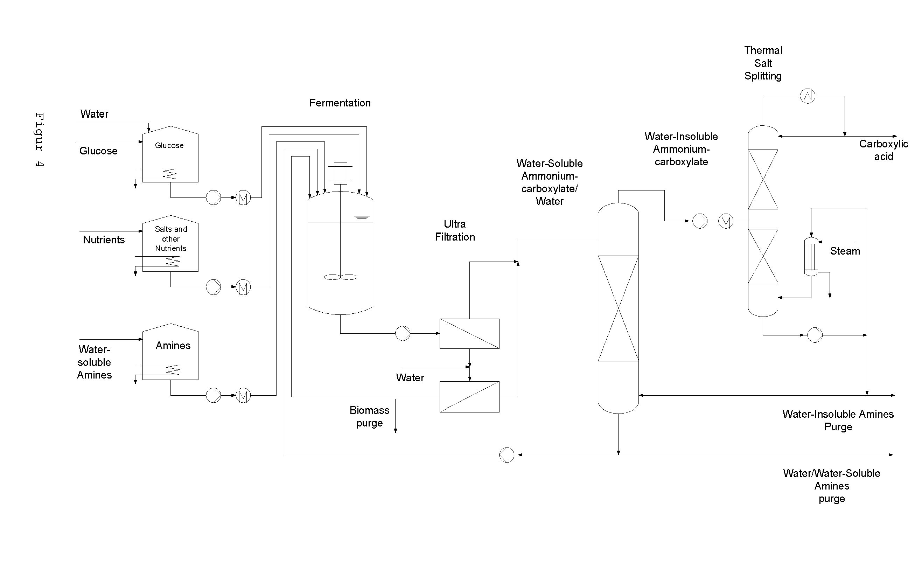

Figur 4 beschreibt eine Ausgestaltung des erfindungsgemäßen Verfahrens, bei welcher in Verfahrensschritt A) ein wasserlösliches Amin zur pH-Wert Regulierung eingesetzt wird. Hierdurch werden in dem Fermenter Ammoniumcarboxylate gebildet. Nach der Biomasseabtrennung werden der wässrigen Lösung wasserunlösliche Amine hinzugegeben. In dieser Verfahrensschritt B) werden die Carboxylate in die organische Phase überführt und gegebenenfalls die wasserunlöslichen Amine teilweise entfernt. Die organische Phase wird der thermischen Salzspaltung zugeführt, wodurch zum einen die freie Säure und zum anderen die Amine gebildet werden. Beide Stoffströme können falls erforderlich in einer separaten Reinigungsstufe gereinigt werden (nicht im Verfahren berücksichtigt). Die so gewonnen Amine können dem Prozess wieder zugeführt werden.

## Patentansprüche

1. Verfahren zur Herstellung von freien α- oder β-Hydroxycarbonsäuren umfassend die Verfahrensschritte
A) Herstellung von α- oder β-Hydroxycarbonsäure durch eine sich in einem wässrigen Medium befindende, biologische Zelle, welche ein Mikroorganismus ist, unter Zugabe eines Amins der allgemeinen Formel (I) 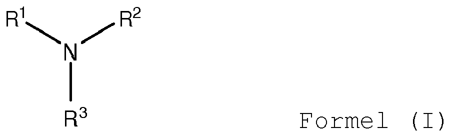 wobei R¹, R² und R³ unabhängig voneinander gleiche oder ungleiche, verzweigte oder unverzweigte, gegebenenfalls substituierte Kohlenwasserstoffreste oder H darstellt,
B) für den Fall, dass das zugegebene Amin in Verfahrensschritt A) wasserlöslich ist, Zugabe eines wasserunlöslichen Amins der allgemeinen Formel (I),
wobei in dem Verfahrensschritt A) beziehungsweise B) ein Mehrphasensystem erhalten wird und sich aus dem wasserunlöslichen Amin und der α- oder β-Hydroxycarbonsäure das korrespondierende Ammoniumcarboxylat bildet, und
C) Abtrennen der wasserunlöslichen Phase und
D) Erhitzen der wasserunlöslichen Phase unter Freisetzung von freier α- oder β-Hydroxycarbonsäure,
**dadurch gekennzeichnet, dass** die α- oder β-Hydroxycarbonsäure ausgewählt ist aus der Gruppe bestehend aus Milchsäure, Zitronensäure, Weinsäure, Glykolsäure, 2-Hydroxyisobuttersäure, 3-Hydroxypropionsäure, 3-Hydroxybuttersäure, 3-Hydroxyvaleriansäure, 3- Hydroxyhexansäure, 3-Hydroxyheptansäure, 3-Hydroxyoctansäure und 3-Hydroxyisobuttersäure,
wobei der Begriff "wasserunlöslich" als eine Löslichkeit von weniger als 100g/kg wässriger Lösung und der Begriff "wasserlöslich" als eine Löslichkeit von gleich oder mehr als 100g/kg wässriger Lösung definiert ist.

2. Verfahren gemäß Anspruch 1, **dadurch gekennzeichnet, dass** in Verfahrensschritt A) ein wasserunlösliches Amin der allgemeinen Formel (I) zugegeben wird.

3. Verfahren gemäß Anspruch 1 oder 2, **dadurch gekennzeichnet, dass** in Verfahrensschritt A) oder B) R¹, R² und R³ unabhängig voneinander gleiche oder ungleiche, unverzweigte, unsubstituierte Alkylreste oder H darstellt.

4. Verfahren gemäß mindestens einem der Ansprüche 1 bis 3, **dadurch gekennzeichnet, dass** in Verfahrensschritt D) die freie α- oder β-Hydroxycarbonsäure unter vermindertem Druck freigesetzt wird.

5. Verfahren gemäß mindestens einem der Ansprüche 1 bis 4, **dadurch gekennzeichnet, dass** in Verfahrensschritt D) die freie α- oder β-Hydroxycarbonsäure in einem Temperaturbereich von 80°C bis 300 °C freigesetzt wird.

6. Verfahren gemäß mindestens einem der Ansprüche 1 bis 5, **dadurch gekennzeichnet, dass** das zugegebene Amin in Verfahrensschritt A) eingesetzt wird, um den pH-Wert des wässrigen Mediums zu beeinflussen.

7. Verfahren gemäß mindestens einem der Ansprüche 1 bis 6, **dadurch gekennzeichnet, dass** in Verfahrensschritt D) die Verbindung der allgemeinen Formel (I) zurück gewonnenen und dem Verfahren wieder zugeführt wird.

8. Verfahren gemäß mindestens einem der Ansprüche 1 bis 7, **dadurch gekennzeichnet, dass** die in Verfahrensschritt A) eingesetzte Zelle ausgewählt ist aus der Gruppe der Gattungen umfassend:
*Aspergillus, Corynebacterium, Brevibacterium, Bacillus, Acinetobacter, Alcaligenes, Lactobacillus, Paracoccus, Lactococcus, Candida, Pichia, Hansenula, Kluyveromyces, Saccharomyces, Escherichia, Zymomonas, Yarrowia, Methylobacterium, Ralstonia, Pseudomonas, Rhodospirillum, Rhodobacter, Burkholderia, Clostridium und Cupriavidus.*

## Claims

1. Process for the preparation of free α- or β-hydroxycarboxylic acids comprising the process steps
A) preparation of α- or β-hydroxycarboxylic acid by a biological cell which is located in an aqueous medium and is a microorganism with addition of an amine of the general formula (I) 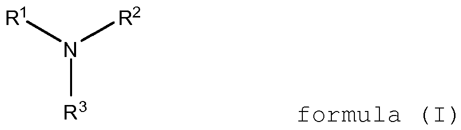 where R¹, R² and R³, independently of one another, are identical or different, branched or unbranched, optionally substituted hydrocarbon radicals or H,
B) for cases where the added amine in process step A) is water-soluble, addition of a water-insoluble amine of the general formula (I),
where, in process step A) and/or B), a multiphase system is obtained and the corresponding ammonium carboxylate is formed from the water-insoluble amine and the α- or β-hydroxycarboxylic acid, and
C) removal of the water-insoluble phase and
D) heating of the water-insoluble phase with
release of free α- or β-hydroxycarboxylic acid **characterized in that** the α- or β-hydroxycarboxylic acid is selected from the group consisting of lactic acid, citric acid, tartaric acid, glycolic acid, 2-hydroxyisobutyric acid, 3-hydroxypropionic acid, 3-hydroxybutyric acid, 3-hydroxyvaleric acid, 3-hydroxyhexanoic acid, 3-hydroxyheptanoic acid, 3-hydroxyoctanoic acid and 3-hydroxyisobutyric acid, wherein the term "water-insoluble" is defined as a solubility of less than 100 g/kg of aqueous solution and the term "water-soluble" is defined as a solubility of equal to or more than 100 g/kg of aqueous solution.

2. Process according to Claim 1, **characterized in that**, in process step A), a water-insoluble amine of the general formula (I) is added.

3. Process according to Claim 1 or 2, **characterized in that**, in process step A) or B), R¹, R² and R³, independently of one another, are identical or different, unbranched, unsubstituted alkyl radicals or H.

4. Process according to at least one of Claims 1 to 3, **characterized in that**, in process step D), the free α- or β-hydroxycarboxylic acid is released under reduced pressure.

5. Process according to at least one of Claims 1 to 4, **characterized in that**, in process step D), the free α- or β-hydroxycarboxylic acid is released in a temperature range from 80°C to 300°C.

6. Process according to at least one of Claims 1 to 5, **characterized in that** the added amine is used in process step A) in order to influence the pH of the aqueous medium.

7. Process according to at least one of Claims 1 to 6, **characterized in that**, in process step D), the compound of the general formula (I) is recovered and returned to the process.

8. Process according to at least one of Claims 1 to 7, **characterized in that** the cell used in process step A) is selected from the group of the genera comprising:
*Aspergillus, Corynebacterium, Brevibacterium, Bacillus, Acinetobacter, Alcaligenes, Lactobacillus, Para coccus, Lactococcus, Candida, Pichia, Hansenula, Kluyveromyces, Saccharomyces, Escherichia, Zymomonas, Yarrowia, Methylobacterium, Ralstonia, Pseudomonas, Rhodospirillum, Rhodobacter, Burkholderia, Clostridium and Cupriavidus.*

## Revendications

1. Procédé pour la préparation d'acides α- ou β-hydroxycarboxyliques libres, comprenant les étapes de processus
A) production d'acide α- ou β-hydroxycarboxylique par une cellule biologique qui est un micro-organisme, se trouvant dans un milieu aqueux, avec addition d'une amine de formule générale (I) 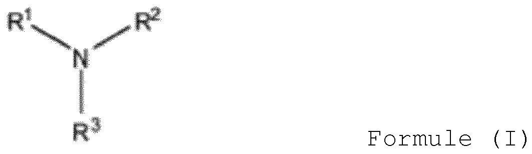 dans laquelle R¹, R² et R³ représentent chacun indépendamment H ou des radicaux hydrocarbonés identiques ou différents, ramifiés ou non ramifiés, éventuellement substitués,
B) dans le cas où dans l'étape A) du procédé l'amine ajoutée est hydrosoluble, addition d'une amine de formule générale (I) insoluble dans l'eau,
avec obtention dans l'étape A) ou respectivement B) du procédé d'un système multiphasique et à partir de l'amine insoluble dans l'eau et de l'acide α- ou β-hydroxycarboxylique se formant le carboxylate d'ammonium correspondant, et
C) séparation de la phase insoluble dans l'eau et
D) chauffage de la phase insoluble dans l'eau avec libération d'acide α- ou β-hydroxycarboxylique libre,
**caractérisé en ce que** l'acide α- ou β-hydroxycarboxylique est choisi dans le groupe constitué par l'acide lactique, l'acide citrique, l'acide d-tartrique, l'acide glycolique, l'acide 2-hydroxy-isobutyrique, l'acide 3-hydroxypropionique, l'acide 3-hydroxybutyrique, l'acide 3-hydroxyvalerique, l'acide 3-hydroxyhexanoïque, l'acide 3-hydroxyheptanoïque, l'acide 3-hydroxyoctanoïque et l'acide 3-hydroxy-isobutyrique,
l'expression « insoluble dans l'eau » étant définie comme une solubilité de moins de 100 g/kg de solution aqueuse et le terme « hydrosoluble » étant défini comme une solubilité égale ou supérieure à 100 g/kg de solution aqueuse.

2. Procédé selon la revendication 1, **caractérisé en ce que** dans l'étape A) du procédé on ajoute une amine de formule générale (I) insoluble dans l'eau.

3. Procédé selon la revendication 1 ou 2, **caractérisé en ce que** dans l'étape A) ou B) du procédé R¹, R² et R³ représentent chacun indépendamment H ou des radicaux alkyle identiques ou différents, non ramifiés, non substitués.

4. Procédé selon au moins l'une quelconque des revendications 1 à 3, **caractérisé en ce que** dans l'étape D) du procédé l'acide α- ou β-hydroxycarboxylique libre est libéré sous pression réduite.

5. Procédé selon au moins l'une quelconque des revendications 1 à 4, **caractérisé en ce que** dans l'étape D) du procédé l'acide α- ou β-hydroxycarboxylique libre est libéré dans une plage de température de 80 °C à 300 °C.

6. Procédé selon au moins l'une quelconque des revendications 1 à 5, **caractérisé en ce que** dans l'étape A) du procédé on utilise l'amine ajoutée afin d'exercer un effet sur le pH du milieu aqueux.

7. Procédé selon au moins l'une quelconque des revendications 1 à 6, **caractérisé en ce que** dans l'étape D) du procédé on récupère le composé de formule générale (I) et on le renvoie dans le processus.

8. Procédé selon au moins l'une quelconque des revendications 1 à 7, **caractérisé en ce que** la cellule utilisée dans l'étape A) du procédé est choisie dans le groupe des genres comprenant :
*Aspergillus, Corynebacterium, Brevibacterium, Bacillus, Acinetobacter, Alcaligenes, Lactobacillus, Paracoccus, Lactococcus, Candida, Pichia, Hansenula, Kluyveromyces, Saccharomyces, Escherichia, Zymomonas, Yarrowia, Methylobacterium, Ralstonia, Pseudomonas, Rhodospirillum, Rhodobacter, Burkholderia, Clostridium* et *Cupriavidus.*
